# EUROPEAN PATENT APPLICATION

(11) **EP 4 623 920 A1**
(43) Date of publication of application: **01.10.2025**
(21) Application number: 25725152.0
(22) Date of filing: 14.02.2025
(51) Int. Cl.: A61K 35/32, A61K 38/39, A61K 47/42, A61K 9/00, A61P 11/02

(54) **PHARMACEUTICAL COMPOSITION COMPRISING INFERIOR TURBINATE STEM CELLS AS ACTIVE INGREDIENT FOR PREVENTING OR TREATING SECONDARY ATROPHIC RHINITIS**

(30) Priority: 16.02.2024 KR 20240022882; 13.02.2025 KR 20250018975
(71) Applicant: The Catholic University Of Korea Industry-Academic Cooperation Foundation, Seocho-gu Seoul 06591 (KR)
(72) Inventor: KIM, Do-Hyun, Seocho-gu, Seoul 06544 (KR); KIM, Sung Won, Gangnam-gu, Seoul 06001 (KR); LIM, Mi-Hyun, Siheung-si, Gyeonggi-do 14994 (KR); PARK, Dan Bi, Seocho-gu, Seoul 06539 (KR); PARK, Sun Hwa, Gwanak-gu, Seoul 08794 (KR); LEE, Hyun Ji, Goyang-si, Gyeonggi-do 10591 (KR); AHN, Min Young, Hanam-si, Gyeonggi-do 12971 (KR); YOO, Seung Yeon, Gangnam-gu, Seoul 06026 (KR)
(74) Representative: Nederlandsch Octrooibureau
(86) International application number: PCT/KR2025/099417
(87) International publication number: WO 2025/174223

(57) **Abstract**

The present invention relates to a pharmaceutical composition for preventing or treating secondary atrophic rhinitis, comprising nasal turbinate-derived stem cells as an active ingredient. The therapeutic efficacy of these stem cells was confirmed when applied to treat secondary atrophic rhinitis, demonstrating excellent treatment effects. The nasal turbinate-derived stem cells of the present invention restored submucosal fibrosis, regenerated cilia, and reversed squamous metaplasia. Moreover, these stem cells remained at the injection site for an extended period, significantly enhancing their therapeutic effects. Given these findings, the present invention is expected to be usefully applied as a preventive or therapeutic agent for secondary atrophic rhinitis.

## Description

### [Technical Field]

The present invention relates to a pharmaceutical composition for the prevention or treatment of secondary atrophic rhinitis, which includes nasal turbinate stem cells as an active ingredient.

This application claims priority to and the benefit of Korean Patent Application No. 10-2024-0122882 filed on February 16, 2024, and Korean Patent Application No. 10-2025-0018975 filed on February 13, 2025. All the contents of these applications, including their specifications and drawings, are incorporated herein by reference.

### [Background Art]

Advancements in biotechnology have opened new possibilities in the fields of cell therapy and regenerative medicine, leading to rapid progress in clinical trials and research for cell therapeutic agents aimed at treating human diseases. Stem cells possess the ability to self-replicate and differentiate into various tissues, making them highly applicable for regenerative medicine, particularly in treating degenerative diseases and severe injuries where conventional treatments are inadequate.

Atrophic rhinitis (AR) is a condition that affects the nasal cavity, occurring when the mucosal tissue and underlying bone shrink, leading to functional changes and various symptoms.

AR is classified into primary and secondary atrophic rhinitis. Primary atrophic rhinitis is known to be caused by viral infections. Patients suffering from secondary atrophic rhinitis experience paradoxical nasal congestion, nasal breathing difficulties, excessive nasal airflow perception, respiratory distress, lung expansion disorders, and subjective sensations of suffocation, significantly impacting their quality of life.

Despite the urgent need for treatments targeting secondary atrophic rhinitis, no therapeutic agent utilizing nasal turbinate stem cells has been reported to date.

### [Disclosure]

### [Technical Problem]

One object of the present invention is to provide a pharmaceutical composition for the prevention or treatment of atrophic rhinitis, comprising nasal turbinate-derived stem cells as an active ingredient.

Another object of the present invention is to provide a cell therapeutic agent for the prevention or treatment of atrophic rhinitis comprising nasal turbinate-derived stem cells as an active ingredient.

A further object of the present invention is to provide a kit for the prevention or treatment of atrophic rhinitis comprising nasal turbinate-derived stem cells and an accompanying instruction manual.

However, the technical problems to be achieved by the present invention are not limited to the aforementioned problems, and other problems that are not mentioned may be clearly understood by those skilled in the art from the following description.

### [Technical Solution]

The present invention provides a pharmaceutical composition for the prevention or treatment of atrophic rhinitis, comprising nasal turbinate-derived stem cells as an active ingredient.

In an exemplary embodiment of the present invention, the nasal turbinate-derived stem cells may be in the form of single cells or spheroids but are not limited thereto.

In another exemplary embodiment of the present invention, the nasal turbinate-derived stem cells may be mixed with type I collagen but are not limited thereto.

In another exemplary embodiment of the present invention, the nasal turbinate-derived stem cells may exhibit differentiation potential into one or more selected from the group consisting of adipocytes, chondrocytes, and osteocytes but are not limited thereto.

In another exemplary embodiment of the present invention, the composition may comprise nasal turbinate-derived stem cells in a concentration ranging from 1×10⁵ to 1×10¹⁰ per mL of type I collagen but is not limited thereto.

In another exemplary embodiment of the present invention, the composition may be an injectable formulation but is not limited thereto.

In another exemplary embodiment of the present invention, the composition may be administered via intranasal injection but is not limited thereto.

In another exemplary embodiment of the present invention, the nasal turbinate-derived stem cells may be human nasal turbinate-derived stem cells (hNTSCs) but are not limited thereto.

In another exemplary embodiment of the present invention, the nasal turbinate-derived stem cells may be negative for at least one marker selected from the group consisting of IgG-PE, CD14, and CD34, or positive for at least one marker selected from the group consisting of CD73, CD90, and CD105 but are not limited thereto.

In another exemplary embodiment of the present invention, the atrophic rhinitis may be secondary atrophic rhinitis but is not limited thereto.

In another exemplary embodiment of the present invention, the composition may be characterized by one or more selected from the group consisting of:
a) Regenerating ciliated cells and increasing the number of ciliated cells;
b) Remaining in the treatment site for an extended period;
c) Restoring squamous metaplasia, submucosal fibrosis, and the pseudo-stratified ciliated columnar epithelium structure;
d) Increasing the thickness of the nasal mucosa;
e) Enhancing the gene expression level of FOXJ1; and
f) Regenerating nasal mucosal tissue.

The present invention provides a cell therapeutic agent for the prevention or treatment of atrophic rhinitis, comprising nasal turbinate-derived stem cells as an active ingredient.

The present invention also provides a kit for the prevention or treatment of atrophic rhinitis, comprising nasal turbinate-derived stem cells and an instruction.

Furthermore, the present invention provides a method for preventing or treating secondary atrophic rhinitis, the method comprising administering a pharmaceutically effective amount of nasal turbinate-derived stem cells or a composition containing same to a subject in need thereof.

Additionally, the present invention provides a use of nasal turbinate-derived stem cells or a composition containing same for the prevention or treatment of secondary atrophic rhinitis.

Furthermore, the present invention provides a use of nasal turbinate-derived stem cells or a composition containing same for the manufacture of a medicament for the prevention or treatment of secondary atrophic rhinitis.

### [Advantageous Effects]

According to the pharmaceutical composition for the prevention or treatment of secondary atrophic rhinitis, comprising nasal turbinate-derived stem cells as an active ingredient, it was confirmed that administering nasal turbinate-derived stem cells for the treatment of secondary atrophic rhinitis exhibited excellent therapeutic effects. The nasal turbinate-derived stem cells of the present invention not only restored submucosal fibrosis and regenerated cilia but also reversed squamous metaplasia. Furthermore, the nasal turbinate-derived stem cells of the present invention were observed to remain at the injection site for an extended period, significantly enhancing these therapeutic effects. Therefore, the nasal turbinate-derived stem cells of the present invention are expected to be usefully applied as an agent for the prevention or treatment of secondary atrophic rhinitis.

### [Description of Drawings]

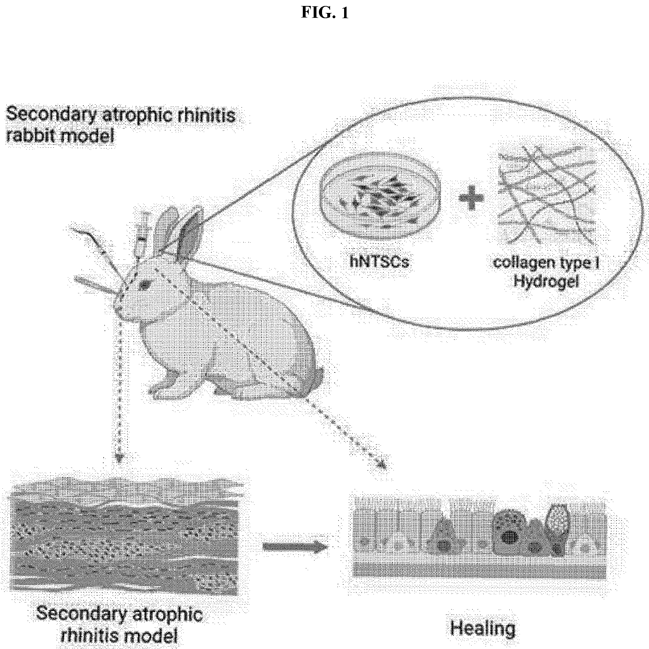

FIG. 1 is a schematic diagram illustrating an experiment to confirm the therapeutic effect of hNTSCs on secondary atrophic rhinitis according to the present invention.
FIG. 2A shows tissue including the surgical site, with the right image depicting the overall tissue morphology captured at 10× magnification (nasal turbinate; NT, septum; S).
FIG. 2B presents the results of H&E staining of the ciliated pseudostratified columnar epithelium in a normal control group and a secondary atrophic rhinitis animal model.
FIG. 2C illustrates squamous metaplasia (arrow), submucosal fibrosis (arrowhead), glandular atrophy (seagull-shaped mark), and residual cell metaplasia (thin arrow), captured at 200× magnification (upper image) and 600× magnification (lower image).
FIG. 2D shows the results of Masson's trichrome (MT) staining of normal control and secondary atrophic rhinitis animal models, confirming the occurrence of submucosal fibrosis in the secondary atrophic rhinitis model.
FIG. 2E (upper row images) displays the results of immunohistochemistry (IHC) analysis identifying the respiratory epithelial cell marker (Acetyl-α-Tubulin), revealing a significant reduction in ciliated cells in the secondary atrophic rhinitis model.
FIG. 2F (lower row images) shows the results of IHC analysis for MUC5AC, captured at 400× magnification in both the normal control group and the secondary atrophic rhinitis animal model.
FIG. 2G presents a graph quantifying the mRNA expression levels of ZEB-1, FOXJ1, KRT14, and MUC5AC in the normal control group and the secondary atrophic rhinitis animal model.
FIG. 3A shows an endoscopic image and microscopic image of the hNTSCs sampled in the present invention.
FIG. 3B presents experimental results confirming the surface markers (IgG-PE, CD14, CD34, CD73, CD90, CD105) of the selected hNTSCs in the present invention.
FIG. 3C shows experimental results verifying the differentiation potential of the selected hNTSCs into adipocytes, chondrocytes, and osteocytes.
FIG. 4A illustrates the spheroids prepared using the selected hNTSCs in the present invention.
FIG. 4B presents experimental results confirming the cell viability of the spheroids, and FIG. 4C shows a graph quantifying the results.
FIG. 4D depicts the injectable formulation prepared using the selected hNTSCs in the present invention.
FIG. 4E shows experimental results evaluating the time-dependent stability of the injectable formulation based on cell viability, and FIG. 4F presents a graph quantifying the results.
FIG. 5A illustrates the surgical procedure for generating the secondary atrophic rhinitis animal model, as well as the processes for creating the Vehicle and Treatment groups.
FIG. 5B presents the therapeutic effects of the injectable formulation using single-cell nasal turbinate-derived stem cells, showing the presence of type I collagen in the injection sites of tissue samples from the Control, Sham, Vehicle, and Treatment groups, as confirmed by MT staining.
FIGS. 5C and 5D demonstrate the therapeutic effects of the injectable formulation using single-cell nasal turbinate-derived stem cells, where ciliary loss was observed in the Vehicle group, whereas ciliary regeneration was confirmed in the Treatment group.
FIG. 5E shows the therapeutic effects of the injectable formulation using single-cell nasal turbinate-derived stem cells, presenting the results of IHC analysis identifying ciliary markers in the Sham, Vehicle, and Treatment groups.
FIG. 5F analyzes the average nasal mucosal thickness in the Sham, Vehicle, and Treatment groups to evaluate the therapeutic effects of the injectable formulation using single-cell nasal turbinate-derived stem cells.
FIGS. 5G and 5H present the therapeutic effects of the injectable formulation using single-cell nasal turbinate-derived stem cells, demonstrating the localization of hNTSCs via HuNu staining and their treatment efficacy in the Treatment group.
FIG. 5I illustrates the therapeutic effects of the injectable formulation using spheroids of nasal turbinate-derived stem cells, showing the mRNA expression levels of FOXJ1 in nasal mucosal tissue from the Sham, Vehicle, and Treatment groups (low, middle, high).
FIG. 5J presents the therapeutic effects of the injectable formulation using spheroids of nasal turbinate-derived stem cells, displaying tissue regeneration effects in the Treatment group as confirmed by H&E and MT staining.

### [Best Mode for Disclosure]

The present invention provides a pharmaceutical composition for the prevention or treatment of atrophic rhinitis, comprising nasal turbinate-derived stem cells as an active ingredient.

The term "stem cell" as used in the present invention refers to a cell that serves as the foundation for cells and tissues constituting an organism, which is capable of self-renewal through repeated division and has the ability to differentiate into cells with specific functions depending on the environment. Stem cells arise in all tissues during the fetal development process and can also be found in certain tissues where cells are actively replaced, such as the bone marrow and epithelial tissues, even in adulthood.

The types of stem cells include embryonic stem cells, which are derived from embryos at an early developmental stage; adult stem cells, which are obtained from cells that constitute tissues after development; and induced pluripotent stem cells, which are generated using gene-editing technology. In this context, adult stem cells are cells present in various tissues of the body and play an important role in maintaining homeostasis. Depending on their tissue of origin, adult stem cells are classified into endoderm-derived tissue stem cells, mesoderm-derived tissue stem cells, and ectoderm-derived tissue stem cells.

Alternatively, stem cells can be classified according to the types of cells they can differentiate into, including totipotent stem cells, which are formed when a fertilized egg undergoes its first division; pluripotent stem cells, which are present in the inner cell mass of the blastocyst and arise from continued division of these cells; and multipotent stem cells, which exist within mature tissues and organs. In this context, multipotent stem cells are cells that can differentiate only into specific cell types characteristic of the tissues and organs in which they are found. They are involved not only in the growth and development of various tissues and organs during the fetal stage, neonatal stage, and adult stage, but also in maintaining homeostasis in adult tissues and inducing regeneration in cases of tissue damage. These tissue-specific multipotent cells are collectively referred to as adult stem cells.

Among adult stem cells, the surgical procedures required to obtain bone marrow-derived stem cells and adipose tissue-derived stem cells cause severe pain and take a considerable amount of time. The amount of stem cells obtained is extremely low, and culturing a clinically sufficient quantity requires significant time and cost. Additionally, there is a high risk of infection and cell loss. In contrast, in the case of human nasal turbinate-derived stem cells, the surgical procedure required for their acquisition involves minimal bleeding and pain and takes less time. Furthermore, stem cells can be continuously secured through the recycling of stem cells isolated from discarded nasal turbinate tissue obtained during nasal turbinate surgery (rhinitis surgery), which is the most frequently performed procedure in the field of otolaryngology. Moreover, the proliferative capacity of nasal turbinate-derived stem cells is higher than that of bone marrow-derived and adipose tissue-derived stem cells.

In one embodiment of the present invention, to confirm the therapeutic use of nasal turbinate-derived stem cells for secondary atrophic rhinitis, nasal turbinate-derived stem cells were administered in the form of single cells or spheroids. In both cases, a therapeutic effect on secondary atrophic rhinitis was confirmed. Accordingly, in one exemplary embodiment of the present invention, the nasal turbinate-derived stem cells may be in the form of single cells or spheroids but are not limited thereto.

In the present invention, the term "spheroid" refers to a three-dimensional cell aggregate capable of self-organization. A spheroid is a small, simplified, three-dimensional organoid that mimics the anatomical structure of actual tissue and can be used for various drug screening applications and disease models. A spheroid generally refers to a three-dimensional structure in which cells are aggregated to the extent that the cross-section appears circular or elliptical. However, the shape should be determined considering the characteristics of the cells or cell aggregates, and it is evident that the term does not necessarily mean a perfect spheroidal or spherical shape.

In the present invention, both single-cell and spheroid forms of nasal turbinate-derived stem cells were mixed with type I collagen. Accordingly, in one exemplary embodiment of the present invention, the nasal turbinate-derived stem cells may be mixed with type I collagen but are not limited thereto. In this context, the term "mixing" may mean that type I collagen serves as a solvent within the composition to achieve an appropriate concentration of single-cell or spheroid-form nasal turbinate-derived stem cells. However, it does not necessarily require a chemical or physical bond between the nasal turbinate-derived stem cells and type I collagen. Furthermore, in the present invention, if the nasal turbinate-derived stem cells are not delivered together with a carrier containing type I collagen, the therapeutic effect on secondary atrophic rhinitis may not be achieved.

In the present invention, "collagen" refers to a major protein of the extracellular matrix (ECM) that provides structural support to the skin, bones, cartilage, and tendons. Collagen is naturally produced in the body and plays a role in maintaining tissue elasticity and strength. Due to these properties, it is widely used in various industries, including cosmetics, medicine, and food, for improving skin elasticity and promoting wound healing.

In this context, type I collagen may be atelocollagen, but is not limited thereto, and any type I collagen with the same function may be applied. In the present invention, the term "atelocollagen" refers to a form of collagen in which the telopeptide, an immunogenic component, has been removed. The atelocollagen of the present invention may include any substance commonly referred to as atelocollagen in the field.

In one exemplary embodiment of the present invention, type I collagen was used as collagen, and the concentration of nasal turbinate-derived stem cells was adjusted using the atelocollagen form of type I collagen. The concentration of the type I collagen used may be from 1% to 10%, for example, from 1% to 9%, from 1% to 8%, from 1% to 7%, from 1% to 6%, from 1% to 5%, from 1% to 4%, from 1% to 3%, from 2% to 10%, from 2% to 9%, from 2% to 8%, from 2% to 7%, from 2% to 6%, from 2% to 5%, from 2% to 4%, from 2% to 3%, from 3% to 10%, from 3% to 9%, from 3% to 8%, from 3% to 7%, from 3% to 6%, from 3% to 5%, from 3% to 4%, or 3%, but is not limited thereto. The above concentration of type I collagen may be prepared using hyaluronic acid as a solvent, but is not limited thereto.

In the present invention, "tri-lineage differentiation potential" refers to the ability of stem cells to differentiate into adipocytes, chondrocytes, and osteocytes. Adipocytes are responsible for energy storage and metabolism regulation, chondrocytes form cartilage tissue and absorb physical shocks, and osteocytes contribute to bone tissue formation and function. The multipotent differentiation potential of stem cells is known to play an important role in tissue damage repair and mobility. In one exemplary embodiment of the present invention, the tri-lineage differentiation potential was analyzed to verify the stemness of nasal turbinate-derived stem cells. As a result, the nasal turbinate-derived stem cells of the present invention were demonstrated to have the potential to differentiate into adipocytes, chondrocytes, and osteocytes. Accordingly, in one exemplary embodiment of the present invention, the nasal turbinate-derived stem cells may have differentiation potential for at least one selected from the group consisting of adipocytes, chondrocytes, and osteocytes, but are not limited thereto.

In one exemplary embodiment of the present invention, the composition may include from 1×10⁵ to 1×10¹⁰ nasal turbinate-derived stem cells per 1 mL of type I collagen, but is not limited thereto.

For example, it may be from 5×10⁵ to 1×10¹⁰, from 6×10⁵ to 1×10¹⁰, from 7×10⁵ to 1×10¹⁰, from 8×10⁵ to 1×10¹⁰, from 9×10⁵ to 1×10¹⁰, from 1×10⁶ to 1×10¹⁰, from 1.5×10⁶ to 1×10¹⁰, from 2×10⁶ to 1×10¹⁰, from 1×10⁵ to 1×10⁹, from 5×10⁵ to 1×10⁹, from 6×10⁵ to 1×10⁹, from 7×10⁵ to 1×10⁹, from 8×10⁵ to 1×10⁹, from 9×10⁵ to 1×10⁹, from 1×10⁶ to 1×10⁹, from 1.5×10⁶ to 1×10⁹, from 2×10⁶ to 1×10⁹, from 1×10⁵ to 1×10⁸, from 5×10⁵ to 1×10⁸, from 6×10⁵ to 1×10⁸, from 7×10⁵ to 1×10⁸, from 8×10⁵ to 1×10⁸, from 9×10⁵ to 1×10⁸, from 1×10⁶ to 1×10⁸, from 1.5×10⁶ to 1×10⁸, or from 2×10⁶ to 1×10⁸, but is not limited thereto.

In the present invention, nasal turbinate-derived stem cells were used in the form of either single cells or spheroids. The number of nasal turbinate-derived stem cells contained in a spheroid was the same as when nasal turbinate-derived stem cells were used in single-cell form, with only differences in cell morphology.

In one exemplary embodiment of the present invention, the composition may be an injectable composition, but is not limited thereto. In one exemplary embodiment of the present invention, the composition may be administered via intranasal injection, but is not limited thereto.

In the present invention, "injection" may refer to the process of delivering the composition to the affected area of secondary atrophic rhinitis via nasal administration. Accordingly, "injectable composition" may include any composition characterized by its formulation, concentration, and density that allows for nasal administration, without being limited to specific examples. In one exemplary embodiment of the present invention, nasal administration was performed by injecting the composition into the nasal mucosa using a syringe. Accordingly, the injectable composition may include an injectable formulation, but is not limited thereto. Furthermore, in the present invention, "intranasal injection" may encompass all administration routes in which the composition is injected into the nasal cavity. In one exemplary embodiment of the present invention, nasal mucosal injection was successfully performed as an example of intranasal injection, but is not limited thereto.

In one exemplary embodiment of the present invention, the nasal turbinate-derived stem cells may be human nasal turbinate-derived stem cells (hNTSCs), but are not limited thereto.

In one exemplary embodiment of the present invention, the nasal turbinate-derived stem cells may be negative for at least one marker selected from the group consisting of IgG-PE, CD14, and CD34, or positive for at least one marker selected from the group consisting of CD73, CD90, and CD105, but are not limited thereto.

In the present invention, CD14 and CD34 may be hematopoietic cell markers, and CD73, CD90, and CD 105 may be mesenchymal stem cell markers, but are not limited thereto.

In one exemplary embodiment of the present invention, the atrophic rhinitis may be secondary atrophic rhinitis, but is not limited thereto.

In one exemplary embodiment of the present invention, the secondary atrophic rhinitis may be caused by turbinate surgery, but is not limited thereto. Secondary atrophic rhinitis is commonly known to result from turbinate surgery, and it has been reported that up to 10% of patients who undergo turbinate surgery develop secondary atrophic rhinitis due to excessive destruction of the inferior turbinate.

In the present invention, secondary atrophic rhinitis may be characterized by localized submucosal fibrosis with spindle-shaped cells, but is not limited thereto.

In one exemplary embodiment of the present invention, the composition may be a pharmaceutical composition characterized by at least one selected from the group consisting of:
a) regenerating ciliated cells and increasing the number of ciliated cells;
b) remaining at the treatment site for an extended period;
c) restoring squamous metaplasia, submucosal fibrosis, and pseudostratified ciliated columnar epithelium;
d) increasing nasal mucosa thickness;
e) increasing the gene expression level of FOXJ1; and
f) regenerating nasal mucosal tissue.

The present invention provides a stem cell therapeutic agent for preventing or treating atrophic rhinitis, including nasal turbinate-derived stem cells as an active ingredient.

In one exemplary embodiment of the present invention, the atrophic rhinitis may be secondary atrophic rhinitis, but is not limited thereto.

In the present invention, "squamous metaplasia" refers to the morphological transformation of squamous epithelial cells due to chronic inflammation or persistent stimulation. Squamous metaplasia may partially resemble squamous cell carcinoma; however, it is known that if the causative factor is removed, the affected cells can revert to their original squamous epithelial form.

In the present invention, "MUC5AC (MUC-5AC)" is a protein encoded by the MUC5AC gene in humans and is a large gel-forming glycoprotein. In the respiratory tract, it binds to inhaled pathogens, which are removed by mucociliary clearance, thereby providing protection against infections. Overproduction of MUC5AC may contribute to diseases such as asthma and chronic obstructive pulmonary disease (COPD), and it has been reported to be associated with enhanced protection against influenza infection.

In the present invention, "ZEB-1 (Zinc finger E-box-binding homeobox 1, ZEB1)" (previously known as TCF8) is a protein encoded by the ZEB1 gene in humans. ZEB-1 is known as a key transcription factor in epithelial-mesenchymal transition (EMT), which is involved in various biological processes such as embryonic development, fibrosis, and tumor progression. It particularly contributes to fibrosis, cancer, and chemoresistance, with fibrosis ultimately leading to cancer progression. Thus, ZEB-1 is recognized in the industry as a biomarker for fibrosis and cancer.

In the present invention, pseudostratified ciliated columnar epithelium refers to the structural composition of tissue, consisting of various cell types, including ciliated cells, goblet cells, and basal cells. In this case, ciliated cells can be identified using acetyl-α-tubulin as a marker, goblet cells using FOXJ1, and basal cells using KRT14. Pseudostratified ciliated columnar epithelium is a type of respiratory epithelium found in the inner lining of the airways, where it allows for air filtration and humidification. In one exemplary embodiment of the present invention, the expression of KRT14 or FOXJ1, which are markers for pseudostratified ciliated columnar epithelium, was found to be reduced. This may indicate the clinical characteristic of dryness observed in secondary atrophic rhinitis.

Since KRT14 is encoded by a gene included in the bronchiolar signature of epithelial cells and can be detected through positive staining, in the present invention, "KRT14" may be used as a biomarker for epithelial cells.

In the present invention, "FOXJ1 (Forkhead Box Protein J1, Forkhead Box J1)" and "Acetyl-α-Tubulin" are transcription factors involved in cilia formation and are known as markers of epithelial cells in the airway. FOXJ1 is located in the nucleus, while acetyl-α-tubulin can be identified by staining non-nuclear structures such as cilia.

The pharmaceutical composition of the present invention may be formulated into various dosage forms, including powders, granules, sustained-release granules, enteric-coated granules, liquids, eye drops, elixirs, emulsions, suspensions, tinctures, troches, aromatic waters, lemonades, tablets, sustained-release tablets, enteric-coated tablets, sublingual tablets, hard capsules, soft capsules, sustained-release capsules, enteric-coated capsules, pills, tinctures, soft extracts, dry extracts, fluid extracts, injections, capsules, perfusion solutions, liniments, lotions, pastes, sprays, inhalants, patches, sterile injectable solutions, or aerosols. The external preparations may be in the form of creams, gels, patches, sprays, ointments, plasters, lotions, liniments, pastes, or cataplasms.

The pharmaceutical composition according to the present invention may include carriers, excipients, and diluents such as lactose, dextrose, sucrose, oligosaccharides, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylparaben, propylparaben, talc, magnesium stearate, and mineral oil.

When formulated, it is typically prepared using diluents or excipients such as fillers, extenders, binders, humectants, disintegrants, and surfactants.

In the pharmaceutical composition of the present invention, additives for tablets, powders, granules, capsules, pills, and troches may include excipients such as corn starch, potato starch, wheat starch, lactose, sucrose, glucose, fructose, D-mannitol, precipitated calcium carbonate, synthetic aluminum silicate, calcium hydrogen phosphate, calcium sulfate, sodium chloride, sodium bicarbonate, purified lanolin, microcrystalline cellulose, dextrin, sodium alginate, methylcellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, Primojel, and the like; binders such as gelatin, gum arabic, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxypropyl methylcellulose, shellac, starch paste, hydroxypropylcellulose, hydroxypropyl methylcellulose, polyvinyl alcohol, polyvinylpyrrolidone, and the like; disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, anhydrous silica, 1-hydroxypropylcellulose, dextran, ion-exchange resins, polyvinyl acetate, formaldehyde-treated casein and gelatin, alginic acid, amylose, guar gum, baking soda, polyvinylpyrrolidone, calcium phosphate, gelled starch, gum arabic, amylopectin, pectin, sodium polyphosphate, ethylcellulose, sucrose, magnesium aluminum silicate, D-sorbitol solution, anhydrous silica, and the like; lubricants such as calcium stearate, magnesium stearate, stearic acid, hydrogenated vegetable oil, talc, clubmoss powder, kaolin, vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, macrogol, synthetic aluminum silicate, anhydrous silica, higher fatty acids, higher alcohols, silicone oil, paraffin oil, polyethylene glycol fatty acid esters, starch, sodium chloride, sodium acetate, sodium oleate, dl-leucine, anhydrous silica, and the like.

The additives for liquid preparations of the present invention may include water, dilute hydrochloric acid, dilute sulfuric acid, sodium citrate, monostearoyl sucrose, polyoxyethylene sorbitol fatty acid esters (Tween esters), polyoxyethylene monoalkyl ethers, lanolin ethers, lanolin esters, acetic acid, hydrochloric acid, ammonia solution, ammonium carbonate, potassium hydroxide, sodium hydroxide, prolamine, polyvinylpyrrolidone, ethylcellulose, and sodium carboxymethylcellulose.

The syrup preparations of the present invention may include a sucrose solution, other sugars, or sweeteners, and may further include, if necessary, flavoring agents, coloring agents, preservatives, stabilizers, suspending agents, emulsifiers, and viscosity-enhancing agents.

The emulsions of the present invention may include purified water, and if necessary, may further include emulsifiers, preservatives, stabilizers, and flavoring agents.

The suspensions of the present invention may include suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, and HPMC 2910, and may further include, if necessary, surfactants, preservatives, stabilizers, coloring agents, and flavoring agents.

The injectable preparations of the present invention may include solvents such as distilled water for injection, 0.9% sodium chloride injection, Ringer's injection, dextrose injection, dextrose + sodium chloride injection, polyethylene glycol (PEG), lactated Ringer's injection, ethanol, propylene glycol, non-volatile oils such as sesame oil, cottonseed oil, peanut oil, soybean oil, corn oil, ethyl oleate, isopropyl myristate, and benzyl benzoate; solubilizing agents such as sodium benzoate, sodium salicylate, sodium acetate, urea, urethane, monoethylacetamide, butazolidin, propylene glycol, Tween series, nicotinamide, hexamine, and dimethylacetamide; buffers such as weak acids and their salts (acetic acid and sodium acetate), weak bases and their salts (ammonia and ammonium acetate), organic compounds, proteins, albumin, peptones, and gums; tonicity agents such as sodium chloride; stabilizers such as sodium bisulfite (NaHSO₃), carbon dioxide gas, sodium metabisulfite (Na₂S₂O₅), sodium sulfite (Na₂SO₃), nitrogen gas (N₂), and ethylenediaminetetraacetic acid (EDTA); antioxidants such as sodium bisulfite 0.1%, sodium formaldehyde sulfoxylate, thiourea, disodium EDTA, and acetone sodium bisulfite; pain-reducing agents such as benzyl alcohol, chlorobutanol, procaine hydrochloride, glucose, and calcium gluconate; and suspending agents such as sodium CMC, sodium alginate, Tween 80, and aluminum monostearate.

The suppositories of the present invention may include bases such as cacao butter, lanolin, Witepsol, polyethylene glycol, glycerogelatin, methylcellulose, sodium carboxymethylcellulose, a mixture of stearic acid and oleic acid, Subanal, cottonseed oil, peanut oil, palm oil, cacao butter + cholesterol, lecithin, lanette wax, monostearin glycerol, Tween or Span, Imhausen, Monolen (monostearoyl propylene glycol), glycerin, Adeps solidus, Butyrum Tego-G, Cebes Pharma 16, Hexaryde Base 95, Cotomar, Hydrokote SP, S-70-XXA, S-70-XX75 (S-70-XX95), Hydrokote 25, Hydrokote 711, Idropostal, Massa Estrarium (A, AS, B, C, D, E, I, T), Massa-MF, Massupol, Massupol-15, Neosupostal-N, Paramount-B, Supposiro (OSI, OSIX, A, B, C, D, H, L), Suppository Base IV type (AB, B, A, BC, BBG, E, BGF, C, D, 299), Suppostal (N, Es), Wecovi (W, R, S, M, Fs), and Tegester Triglyceride Base (TG-95, MA, 57).

Solid formulations for oral administration include tablets, pills, powders, granules, and capsules. These solid formulations are prepared by mixing the above-mentioned extract with at least one excipient, such as starch, calcium carbonate, sucrose, lactose, or gelatin. In addition to simple excipients, lubricants such as magnesium stearate and talc may also be used.

Liquid formulations for oral administration include suspensions, solutions, emulsions, and syrups. These liquid formulations may contain commonly used simple diluents such as water and liquid paraffin, as well as various excipients such as humectants, sweeteners, flavoring agents, and preservatives. Formulations for parenteral administration include sterile aqueous solutions, nonaqueous solvents, suspensions, emulsions, freeze-dried preparations, and suppositories. Nonaqueous solvents and suspensions may include propylene glycol, polyethylene glycol, vegetable oils such as olive oil, and injectable esters such as ethyl oleate.

The pharmaceutical composition according to the present invention is administered in a pharmaceutically effective amount. In the present invention, a "pharmaceutically effective amount" refers to an amount sufficient to treat the disease with a reasonable benefit/risk ratio applicable to medical treatment. The effective dosage level may be determined based on factors such as the type and severity of the patient's disease, drug activity, drug sensitivity, administration time, administration route, excretion rate, treatment period, concomitant medications, and other factors well known in the medical field.

The pharmaceutical composition according to the present invention may be administered as an individual therapeutic agent or in combination with other therapeutic agents. It may be administered sequentially or simultaneously with conventional therapeutic agents and may be administered as a single or multiple doses. It is important to administer the minimum amount necessary to achieve maximum efficacy without side effects, which can be easily determined by a person skilled in the art to which the present invention pertains.

The pharmaceutical composition of the present invention may be administered to a subject via various routes. All routes of administration are conceivable, including, for example, oral administration, subcutaneous injection, intraperitoneal administration, intravenous injection, intramuscular injection, perispinal (epidural) injection, sublingual administration, buccal administration, rectal insertion, vaginal insertion, ocular administration, auricular administration, nasal administration, inhalation, spraying into the mouth or nose, topical administration, and transdermal administration.

The pharmaceutical composition of the present invention is determined based on various factors, including the disease to be treated, the route of administration, the age, sex, weight, and severity of the disease in the patient, along with the type of active ingredient.

In the present invention, the term "subject" refers to an entity requiring treatment for a disease and, more specifically, may include humans or non-human primates, mice (Mus musculus), rats (Rattus norvegicus), dogs, cats, horses, and cattle, among other mammals.

In the present invention, "administration" refers to providing the specified pharmaceutical composition of the present invention to a subject by any appropriate method.

In the present invention, "prevention" refers to all actions that suppress or delay the onset of the target disease, while "treatment" refers to all actions in which administration of the pharmaceutical composition according to the present invention improves or beneficially modifies the target disease and its associated metabolic abnormalities. "Improvement" refers to all actions that reduce disease-related parameters, such as symptom severity, through the administration of the composition according to the present invention.

The present invention provides a stem cell therapeutic agent for preventing or treating atrophic rhinitis, comprising nasal turbinate-derived stem cells as an active ingredient.

In the present invention, the term "cell therapeutic agent" refers to a therapeutic agent that utilizes autologous, allogenic, or xenogenic cells to restore tissue function. In the present invention, it refers to a therapeutic agent that uses animal cells to regenerate damaged tissue. In the present invention, the term "stem cell therapeutic agent" may refer to a cell therapeutic agent in which the cells used are stem cells.

The present invention provides a kit for preventing or treating atrophic rhinitis, comprising nasal turbinate-derived stem cells and an instruction manual.

In one embodiment of the present invention, the atrophic rhinitis may be secondary atrophic rhinitis, but is not limited thereto.

In the present invention, the term "kit" refers to a tool that enables the prevention or treatment of secondary atrophic rhinitis using nasal turbinate-derived stem cells of the present invention. In addition to the above-described material, the kit may further include other components, compositions, solutions, or devices that are conventionally required for storage and processing. For example, each component may be applied one or more times without limitation, and there is no restriction on the order of application of each material. The application of each material may proceed simultaneously or separately.

In the present invention, the kit may include a container, an instruction manual, and the like. The container may serve the function of packaging the above-described material, as well as storing and securing it. The container may take various forms, such as a bottle, tub, small sachet, envelope, tube, or ampoule, and may be made partially or entirely of plastic, glass, paper, foil, wax, or other materials. Initially, the container may have a lid that is either a part of the container itself or attached mechanically, adhesively, or by other means, which may be fully or partially detachable. Additionally, the container may be equipped with a stopper that allows access to the contents using a syringe needle. The kit may also include an outer package, which may contain instructions regarding the use of its components.

Furthermore, the present invention provides a method for preventing or treating secondary atrophic rhinitis, comprising the step of administering a pharmaceutically effective amount of nasal turbinate-derived stem cells or a composition containing the same as an active ingredient to a subject in need thereof.

Furthermore, the present invention provides a use of nasal turbinate-derived stem cells or a composition containing the same as an active ingredient for preventing or treating secondary atrophic rhinitis.

Furthermore, the present invention provides a use of nasal turbinate-derived stem cells or a composition containing the same as an active ingredient for the manufacture of a medicament for preventing or treating secondary atrophic rhinitis.

### [Mode for Disclosure]

Hereinafter, a preferred embodiment is presented to aid understanding of the present invention. However, the following examples are provided to more easily understand the present invention, and the content of the present invention is not limited by the following examples.

### [Example]

### Preparation of White Rabbits for Animal Model

New Zealand White rabbits (SPF NZW; Kangda, Qingdao, China) were divided into four groups: a model group that underwent turbinate surgery, a sham group that underwent surgery with type I collagen injection during surgery, a treatment group that underwent surgery with hNTSC application, and a normal control group (NC) that did not undergo any intervention. All procedures complied with relevant ethical guidelines, including approval from the Institutional Animal Care and Use Committee (IACUC) of The Catholic University (CUMS-2022-0289-03).

### Isolation and Culture of hNTSCs from Human Inferior Turbinate Tissue

Fresh hNTSCs were sampled from the inferior turbinate tissue of a 22-year-old male. This procedure was conducted at Seoul St. Mary's Hospital and received ethical approval from the Institutional Review Board of The Catholic University (KC18TESI0167). Initially, the tissue was rinsed with gentamicin solution in the operating room and subsequently washed with an antibiotic-antifungal solution (Gibco, Gaithersburg, MD, USA) and Dulbecco's phosphate-buffered saline (D-PBS, Welgene, Namcheon, South Korea).

The tissue was then cut into fragments with a thickness of 0.5 mm³ and cultured at 37°C in α-MEM (Minimum Essential Medium Eagle - alpha modification) supplemented with 10% fetal bovine serum. The culture environment was maintained with 5% CO₂ and sealed with a sterilized glass slide.

After a 2-week culture period, the cells were harvested from the plate and subsequently dissociated using a 1 mL solution of 0.25% trypsin/1 mM ethylenediaminetetraacetic acid (EDTA). The cell morphology and concentration were examined under 40× magnification.

### Flow Cytometric Analysis

A single-cell suspension of hNTSCs was prepared and incubated with phycoerythrin-conjugated antibodies specifically targeting mesenchymal stem cell surface markers, including CD14, CD34, CD73, CD90, and CD105 (BD Pharmingen, San Diego, CA, USA). After a 1-hour incubation, the cells were washed and resuspended in D-PBS.

### Preparation of Therapeutic Agents

For the formulation of the therapeutic agent administered to the treatment group, hNTSCs were harvested from culture dishes and pelleted. The cells were then mixed with cold type I collagen (COLTRIX^{®} TendoRegen, Ubiosis, Sungnam, South Korea) at a concentration of 2 × 10⁶ cells/mL. Using a 1-mL insulin syringe with a 29-gauge needle (BD Pharmingen), an exact volume of 100 µL was administered. Similarly, for the preparation of the sham group, 100 µL of type I collagen was injected to ensure procedural uniformity.

### Surgical Procedure

To establish a secondary atrophic rhinitis (empty nose syndrome, ENS) animal model using rabbits, the bone flap of the nasal dorsum was first removed. A 3 cm incision was made along the midline from the nasal tip, and the skin flap and periosteal flap were carefully dissected. Then, a micro handpiece equipped with a disc burr was used to resect a 0.5 × 2.5 cm rectangular section on both sides of the nasal septum. The excised bone flap was preserved in saline. To create a nasal turbinate defect, bovie bipolar forceps were used to perform electrocauterization. After creating the defect, a collagen type I hydrogel (COLTRIX^{®} TendoRegen) mixture containing 2 × 10⁶ cells/mL was prepared, and 100 µL was injected into one side of the nasal turbinate. The bone flap preserved in saline was repositioned, and the periosteum and periosteal flap were sutured to complete the procedure. The rabbits were observed for **two months** post-surgery.

### Tissue Processing

The rabbits were euthanized using potassium chloride. Samples were fixed overnight in 4% paraformaldehyde and rinsed with phosphate-buffered saline (PBS). Decalcification was performed in 1% ethylenediaminetetraacetic acid (EDTA, pH 8) for two months, followed by paraffin embedding.

### Histological Analysis

Tissue sections of 4.5 µm thickness were deparaffinized in xylene and rehydrated through graded ethanol. Hematoxylin (Abcam, Cambridge, UK) and Eosin-Y (Siheung, South Korea) staining were performed along with Masson's trichrome staining (Abcam, Cambridge, UK) for collagen visualization.

### Immunohistochemistry (IHC) and Immunofluorescence (IF) Analysis

Tissue sections underwent epitope retrieval in 0.01 M citrate buffer (Sigma-Aldrich, St. Louis, MO, USA) for 5 minutes. Endogenous peroxidase activity was quenched using 3% hydrogen peroxide (H₂O₂). Sections were then blocked with 5% normal goat serum (Vector Laboratories, Burlingame, CA, USA) for 1 hour.

For IHC, primary antibodies against Mucin5AC (1:100; Abcam) and acetylated-α-tubulin (1:100; Santa Cruz Biotechnology, Dallas, TX, USA) were applied for 2 hours. The sections were washed with PBS and treated with a mixture of anti-rabbit and anti-mouse horseradish peroxidase (HRP) polymers (GBI Labs, Bothell, WA, USA) for 30 minutes. Staining was performed using diaminobenzidine (DAB; GBI Labs), followed by hematoxylin counterstaining.

For IF analysis, sections were incubated with the primary antibody anti-HuNu (1:200; Sigma-Aldrich) for 2 hours, washed, and then treated with the secondary antibody goat anti-mouse IgG Alexa Fluor^{™} 555 (1: 1000; Invitrogen, Carlsbad, CA, USA) for 1 hour. Counterstaining was performed using DAPI (1 µg/mL; Invitrogen). The slides were examined using a confocal laser microscope (LSM800; Carl Zeiss, Oberkochen, Germany).

### RNA Extraction and Quantitative Real-Time Polymerase Chain Reaction (qRT-PCR)

Total RNA was extracted from unfixed tissue using TRIzol (Thermo Fisher Scientific, Waltham, MA, USA). Reverse transcription was performed using iScript^{™} (Bio-Rad, Hercules, CA, USA), followed by amplification using the CFX96^{™} system (Bio-Rad). Relative mRNA levels were calculated using the 2-ΔΔCt method with HPRT1 as the reference gene. The primer sequences are listed in Table 1.

**[Table 1]**

| **NO** . | **NAME** | **SEQUENCE** |
|---|---|---|
| 1 | HPRT1_F | GAC CAG TCA ACA GGG GAC AT |
| 2 | HPRT1_R | CTT GCG ACC TTG ACC ATC TT |
| 3 | FOXJ1_F | TCG ACT GGG AAG CCA TCT |
| 4 | FOXJ1_R | GTC GAA GTC CAG GCT GTT G |
| 5 | Keratin 14_F | GAA GGA GGA ACT GGC CTA CC |
| 6 | Keratin 14_R | TCT CGT ACT GGT CAC GCA TC |
| 7 | MUC5AC_F | TCT GCT GTC CCG AGA GAA CAC |
| 8 | MUC5AC_R | CTG CCA TCA CAA ATG ACC AC |
| 9 | ZEB-1_F | GCC TAC AGA ACC CAA CTG GA |
| 10 | ZEB-1_R | TCT CTC CGC TGT GAA TCC TT |

### Statistical Analysis

All statistical evaluations were performed using a two-way analysis of variance (ANOVA) with Prism 5.0 software (GraphPad Software, San Diego, CA, USA).

### Example 1. Confirmation of Histological Changes in the Secondary Atrophic Rhinitis Model

### Example 1-1. Validation of the Secondary Atrophic Rhinitis Animal Model via H&E Staining

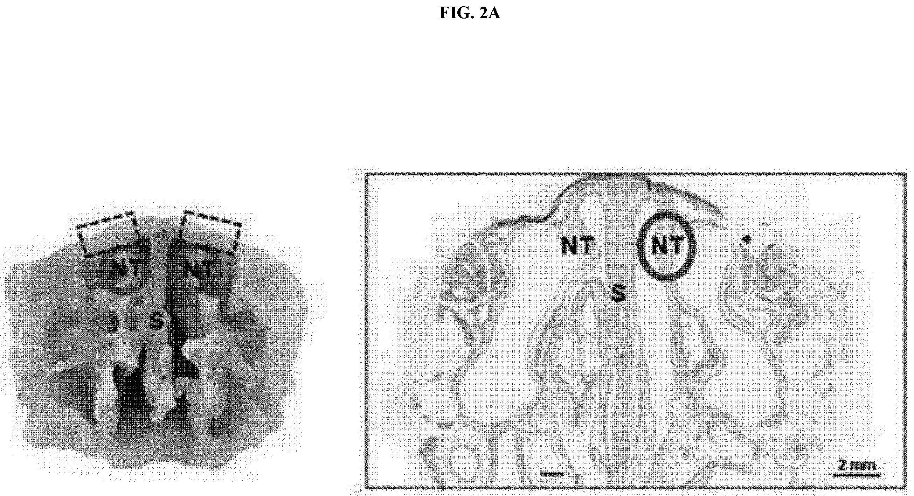

To validate the secondary atrophic rhinitis animal model developed in this study, complete nasal cavity extraction through a conical sectioning method was performed (Figure 2A). Subsequently, histological analysis using hematoxylin and eosin (H&E) staining was conducted to confirm whether the model reflects the histological characteristics of secondary atrophic rhinitis. The normal control (NC) group was used as the control.

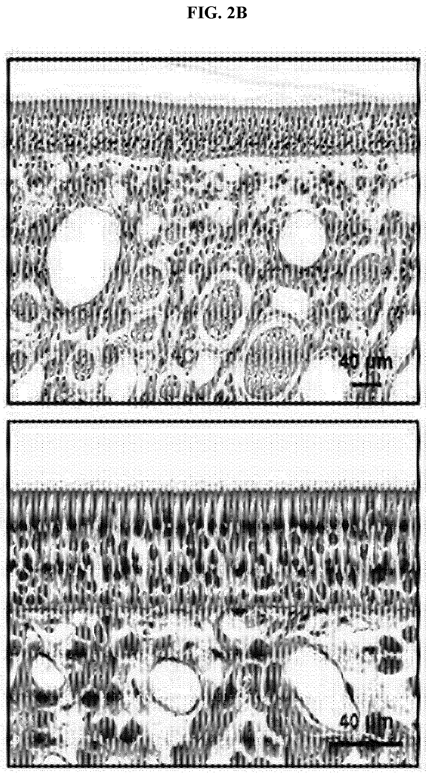

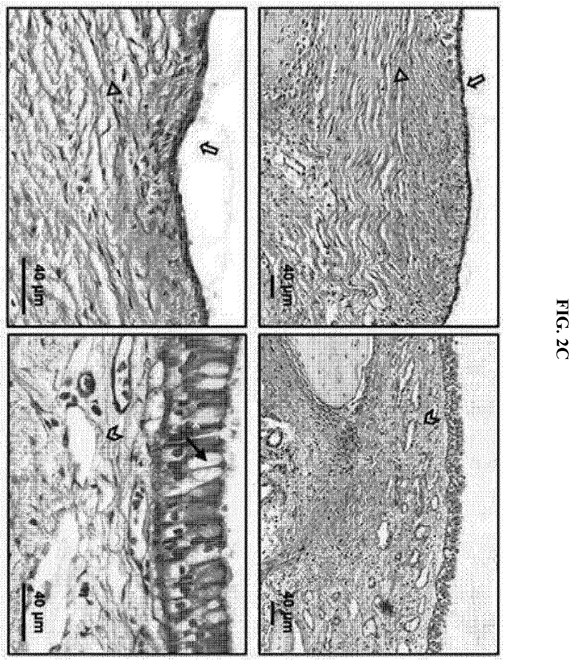

As a result, the ciliated pseudostratified columnar epithelium of the normal control group and the secondary atrophic rhinitis animal model was identified as shown in Figure 2B. Additionally, squamous cell metaplasia (arrows), submucosal fibrosis (arrowheads), glandular atrophy (seagull shapes), and goblet cell metaplasia (thin arrows) were observed in both the normal control group and the secondary atrophic rhinitis animal model, as shown in Figure 2C. A comparative evaluation of histological parameters for secondary atrophic rhinitis in both groups is presented in Table 2.

**[Table 2]**

| **Variables** | **Normal Controls,** n/N (%) | **Models,** n/N (%) |
|---|---|---|
| Squamous cell metaplasia | 0/3 (0%) | 2/3 (66.7%) |
| Submucosal fibrosis | 0/3 (0%) | 3/3 (100%) |
| Glandular atrophy | 0/3 (0%) | 3/3 (100%) |
| Goblet cell metaplasia | 0/3 (0%) | 2/3 (66.7%) |

These results indicate that the normal control group exhibited well-preserved pseudostratified epithelium with intact cilia. In contrast, the secondary atrophic rhinitis model group displayed significant histological changes characteristic of secondary atrophic rhinitis, including squamous cell metaplasia, submucosal fibrosis, glandular atrophy, and goblet cell metaplasia. Notably, as shown in Figure 2B, areas with pronounced submucosal fibrosis exhibited a complete reduction in glandular structures.

### Example 1-2. Validation of the Secondary Atrophic Rhinitis Animal Model Based on Marker Analysis

To elucidate the histological variations associated with secondary atrophic rhinitis, specific marker analyses were performed. Marker analysis was conducted using Masson's trichrome (MT) staining and immunohistochemical (IHC) analysis.

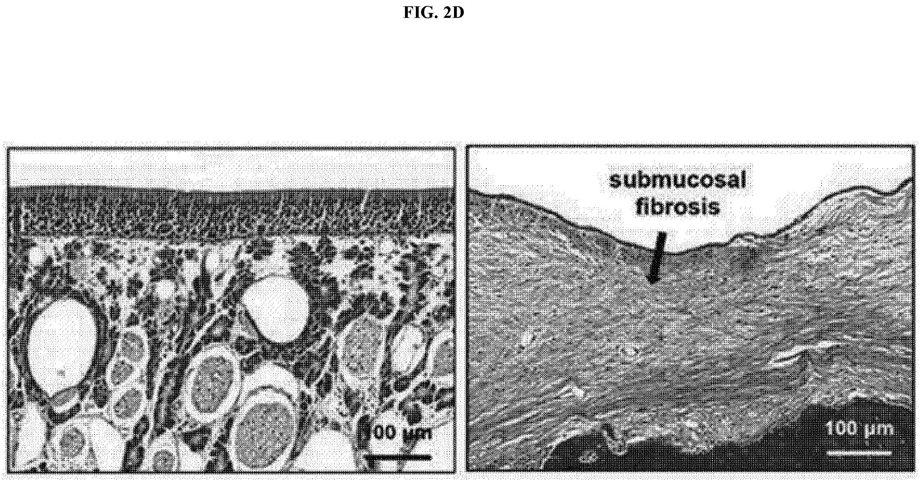

Masson's trichrome (MT) staining was used to visualize collagen fibrosis, which appeared as blue-stained regions. The results indicated that high concentrations of collagen were localized within fibrotic tissues. Comparative analysis between the normal control group and the secondary atrophic rhinitis model demonstrated the presence of fibrosis in the submucosal layer of the secondary atrophic rhinitis model, as indicated by the blue staining (Figure 2D).

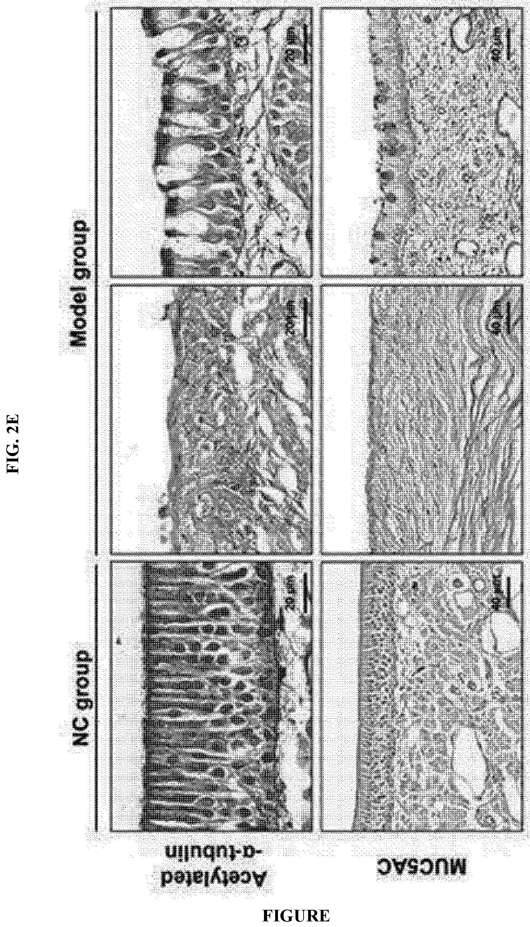

IHC analysis was utilized to identify markers of respiratory epithelial cells. Ciliated cells were targeted using an antibody against acetylated-α-tubulin. The presence of ciliated cells was sporadic, but a significant reduction was observed in the secondary atrophic rhinitis model (Figure 2E).

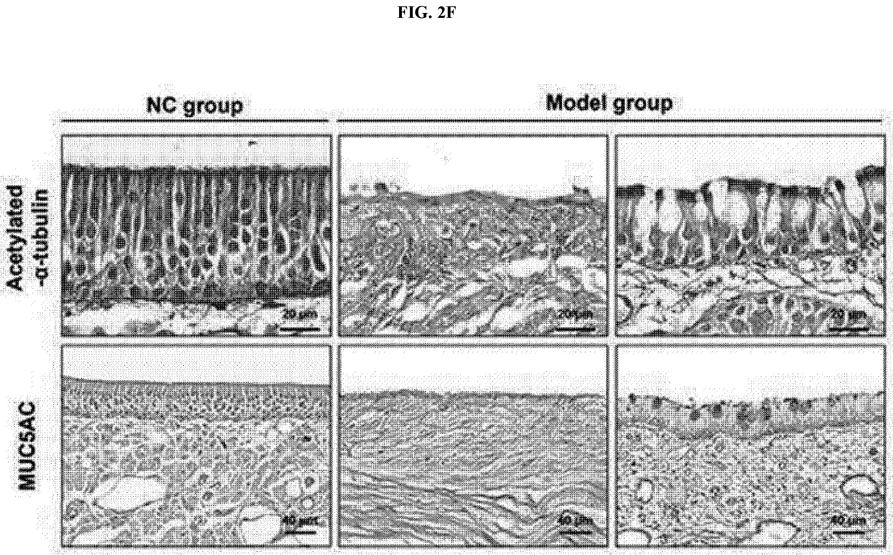

MUC5AC staining revealed two distinct expression patterns in goblet cells. In areas where pseudostratified columnar structures were degraded due to squamous cell metaplasia, MUC5AC expression was notably absent. However, in regions exhibiting goblet cell metaplasia, MUC5AC expression was concentrated (Figure 2F).

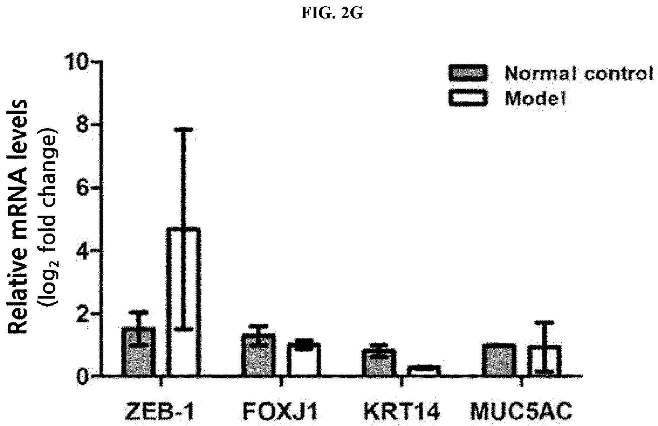

Additionally, upregulation of the fibrosis marker Zinc Finger E-Box Binding Homeobox 1 (ZEB-1) was observed. Apart from MUC5AC, markers associated with ciliated pseudostratified columnar epithelium, such as KRT14 and FOXJ1, showed reduced expression. However, the differences between the two groups did not reach statistical significance (Figure 2G).

### Example 2. Selection and Differentiation Potential Verification of Turbinate-Derived Stem Cells

### Example 2-1. Selection of Turbinate-Derived Stem Cells

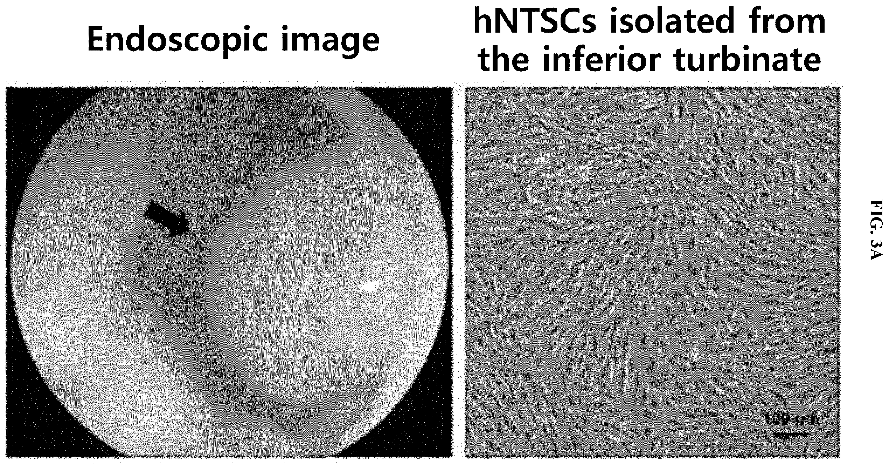

The hNTSCs sampled according to the experimental method of this invention are shown in Figure 3A. To evaluate the cellular composition of the collected hNTSCs, flow cytometric analysis was performed using a BD FACS Canto II (Becton Dickinson, Franklin Lakes, NJ, USA).

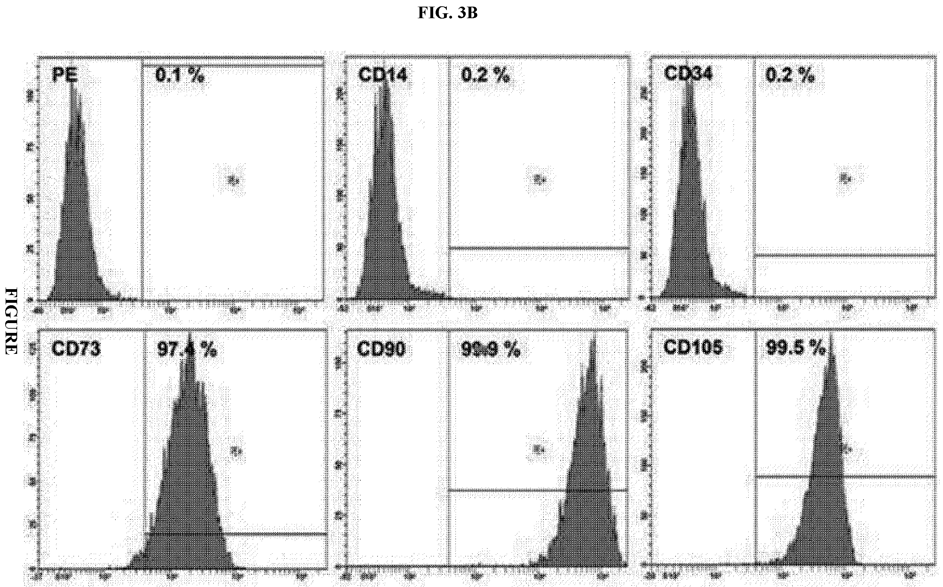

The results demonstrated that the hNTSCs used in this invention were selected based on confirmed negative for the hematopoietic cell markers CD14 and CD34 and positive for the mesenchymal stem cell markers CD73, CD90, and CD105, as described in Figure 3B.

### Example 2-2. Verification of the Differentiation Potential of Turbinate-Derived Stem Cells

To verify the turbinate-derived stem cells selected in Example 2-1, the tri-lineage differentiation potential into adipocytes, chondrocytes, and osteocytes was evaluated using passage 6 hNTSCs. For adipogenic differentiation, hNTSCs were seeded into a 4-well plate at a density of 1.5 × 10⁴ cells per well, and the culture medium was replaced with StemPro^{™} Adipogenesis Differentiation Kit (Gibco). After two weeks, cells were fixed with 2% paraformaldehyde (PFA) and stained with Oil Red O (Sigma-Aldrich, St. Louis, MO, USA) to assess adipogenic differentiation. For chondrogenic differentiation, hNTSCs were first aggregated into spheroids using the hanging-drop method for two days. The formed spheroids were then transferred into StemPro^{™} Chondrogenesis Differentiation Medium (Gibco). After two weeks, the spheroids were fixed with 2% PFA, embedded in paraffin, and sectioned into 4.5 µm thick slices. The sections were deparaffinized with xylene, rehydrated through graded ethanol series, and stained with Alcian Blue (Abcam) to assess chondrogenic differentiation. For osteogenic differentiation, hNTSCs were cultured in StemPro^{™} Osteogenesis Differentiation Kit (Gibco) to induce differentiation into the osteogenic lineage. After three weeks, the cells were fixed with 2% PFA and stained with 2% Alizarin Red (Sigma-Aldrich) to confirm calcium deposition, indicative of osteogenic differentiation.

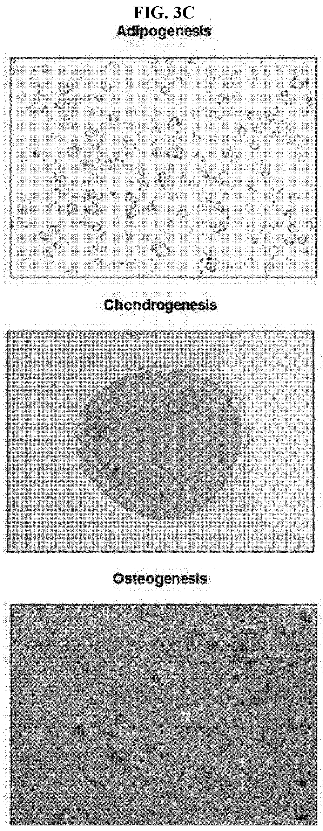

The results confirmed that the turbinate-derived stem cells in this invention exhibited multipotency, successfully differentiating into adipocytes, chondrocytes, and osteocytes (Figure 3C).

### Example 3. Preparation of Spheroids and Injectable Formulations Using Human Nasal Turbinate-Derived Stem Cells

### Example 3-1. Preparation of Spheroids Using Human Nasal Turbinate-Derived Stem Cells and Confirmation of Their Viability

Spheroids (three-dimensional cell aggregates) were prepared using the human nasal turbinate-derived stem cells (hNTSCs) verified in Example 2. The spheroids were generated using the StemFIT 3D^{®} plate (H853400, Microfit, Gyeonggi, Korea).

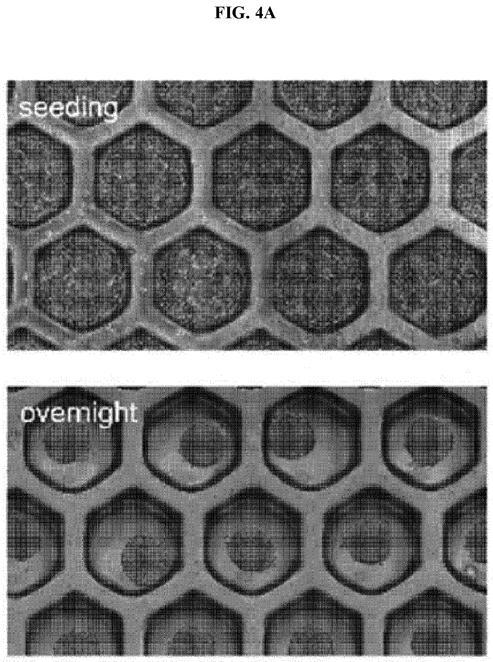

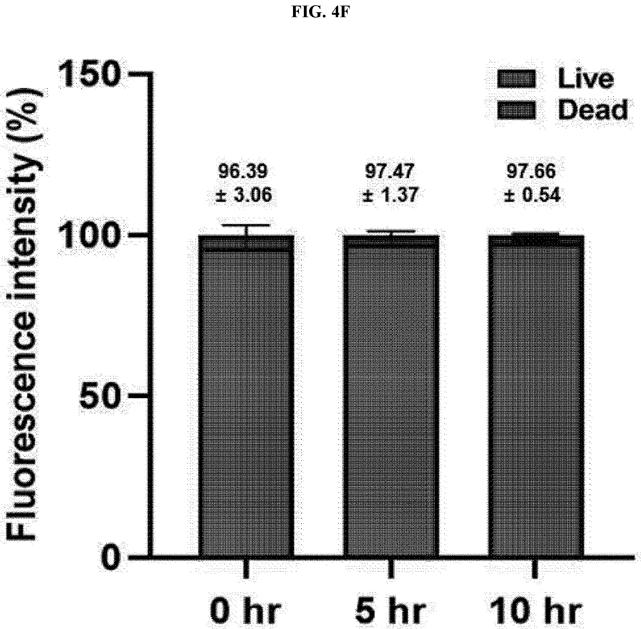

As a result, the spheroids exhibited a size of 251.6 ± 10.4 µm, with an average diameter of 250 µm, confirming that the method of the present invention enables the formation of uniformly sized spheroids (Figure 4A).

Next, the safety of the spheroids was evaluated by assessing cell viability. To do this, 1.5 × 10⁶ cells were seeded into a StemFIT grid-patterned well. After overnight incubation (14-16 hours), spheroid formation was confirmed, and the spheroids were collected for viability analysis. The spheroids were stained with Calcein-AM (green, live) and EthD-1 (red, dead) to assess cell viability.

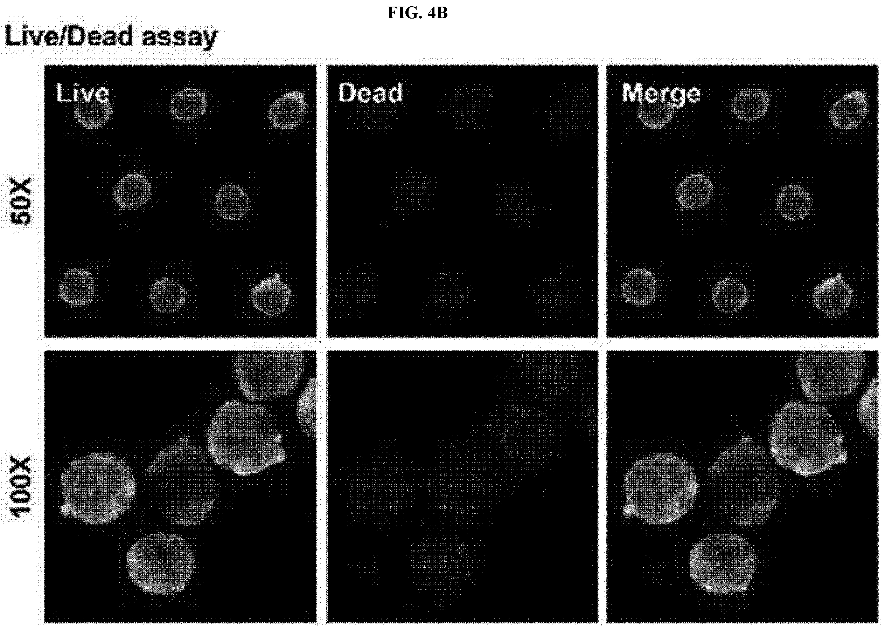

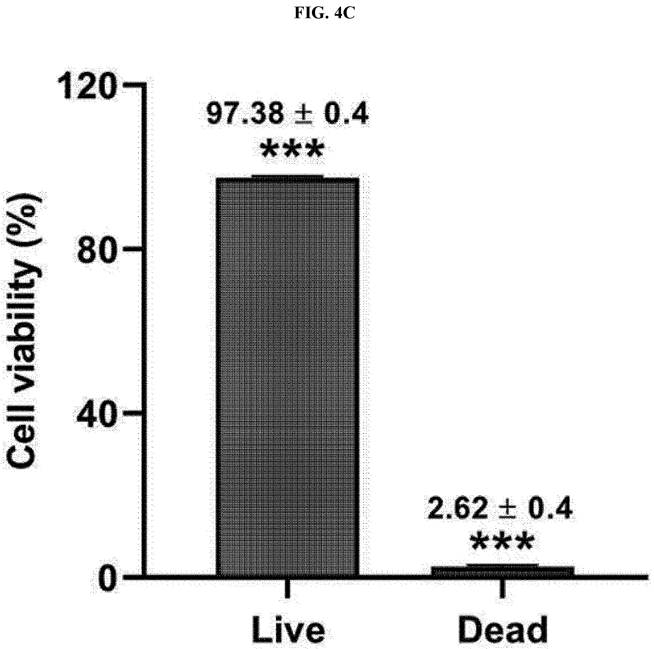

As a result, 98% of the total spheroid cells were viable, demonstrating remarkably high cell viability (Figure 4B and Figure 4C). High cell viability indicates that the cells used for treatment can effectively maintain their function in vivo while minimizing side effects. Therefore, this experimental result suggests that hNTSCs of the present invention exhibit excellent safety as a cell therapeutic agent.

### Example 3-2. Preparation and Confirmation of Injectable Formulations Using Human Nasal Turbinate Stem Cells

Injectable formulations were prepared using the spheroids of human nasal turbinate stem cells manufactured in Example 3-1. In this step, hNTSCs alone and spheroids were each mixed with collagen type I hydrogel (COLTRIX^{®} TendoRegen) as a supporting scaffold, thus developing an advanced bio-hybrid injectable formulation. Specifically, after seeding cells into the StemFIT plate, the resulting spheroids were collected using pipetting or scraping with a tip. The spheroids were then gently centrifuged (200 g, 3 min) to collect them at the bottom of the tube, and the medium was removed. Then, collagen type I was added at the appropriate concentration. To adjust the concentration, the following method was applied: For instance, since one StemFIT product yields 1.5 × 10^6 hNTSCs, to achieve a low concentration group of 2 × 10^6 cells/mL, 750 µL of collagen type I was added to the tube containing spheroids and mixed by pipetting to achieve the target concentration. For the injectable formulation, an insulin syringe (30G) was used to minimize injury to the nasal mucosa during injection into the nasal cavity (Figure 4C). The thoroughly mixed formulation was stored in the refrigerator in single-dose syringes.

Next, the stability of the prepared injectable formulation was evaluated by analyzing cell viability over time. Specifically, the injectable formulation containing collagen type I and spheroids was stained with Calcein-AM (green-live) and EthD-1 (red-dead), and cell viability was analyzed at 5 and 10 hours post-preparation.

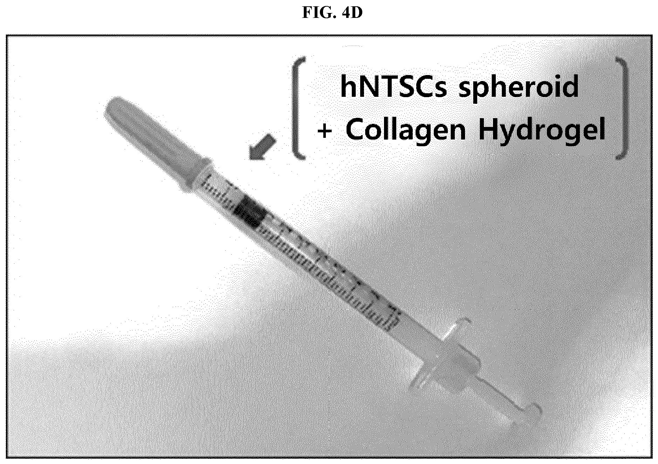

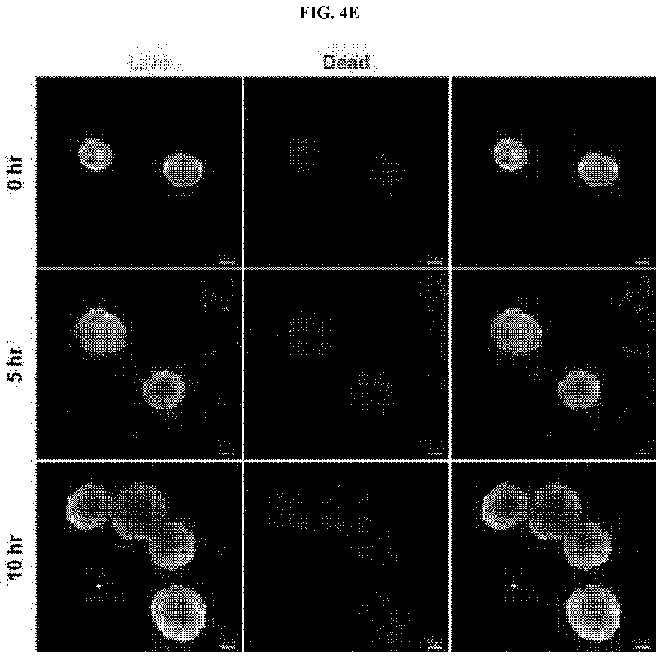

As a result, the injectable formulation incorporating spheroids exhibited excellent cell viability even after 10 hours, confirming its stability as an injectable formulation (Figures 4D and 4E). Considering that a time delay between the preparation of the injectable formulation and its administration into the nasal mucosa is inevitable due to transportation and surgical procedures, these results suggest that the injectable formulation remains stable and retains therapeutic activity for treating secondary atrophic rhinitis.

### Example 4. Confirmation of the Therapeutic Effect of Human Nasal Turbinate Stem Cells on Secondary Atrophic Rhinitis

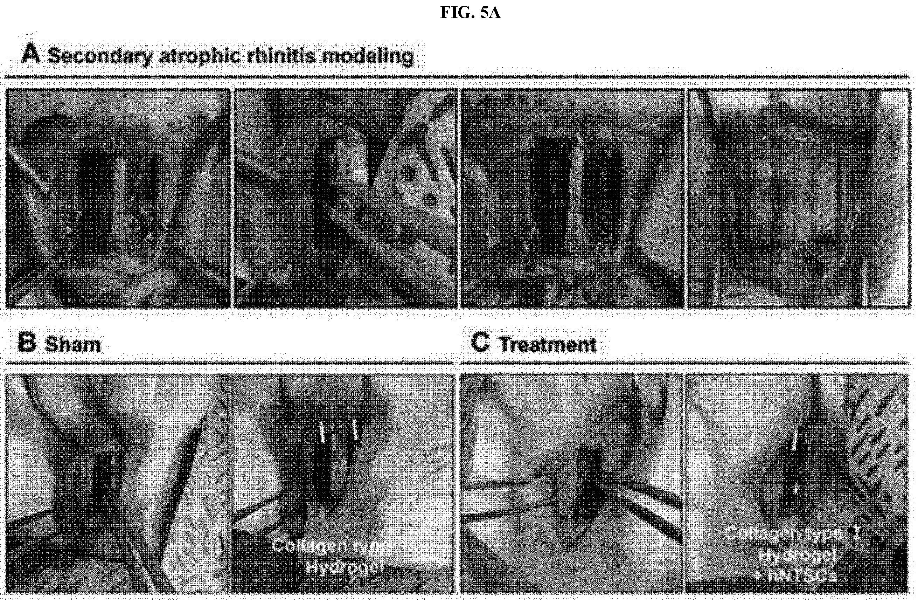

Following the experimental method of this invention and Figure 5A, the secondary atrophic rhinitis model developed in Example 1 was used to evaluate the model group, sham group, and treatment group. For the treatment group, 50 µL of the injectable formulation was administered to the electrocauterized area at the following concentrations: Low concentration: 2 × 10^6 cells/mL, Medium concentration: 1 × 10^7 cells/mL, High concentration: 1 × 10^8 cells/mL The injectable formulation with single-cell hNTSCs was administered at a low concentration, while the Vehicle group received the same amount of collagen type I (COLTRIX^{®} TendoRegen) at the electrocauterized area.

### Example 4-1. Confirmation of the Therapeutic Effect of Single-Cell Injection of Human Nasal Turbinate Stem Cells in Secondary Atrophic Rhinitis

First, Masson's trichrome (MT) staining was performed after injecting the respective formulations into the Control, Sham, Vehicle, and Treatment groups.

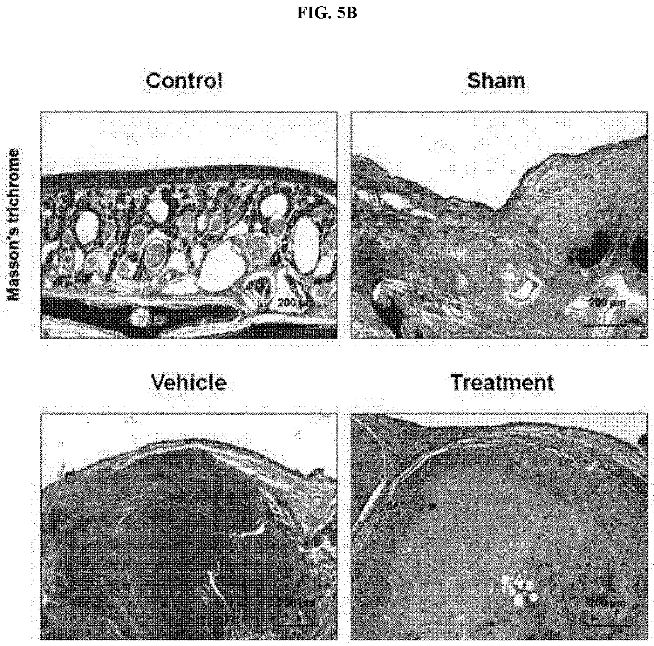

As a result, collagen type I was confirmed at the injection site within the tissue, indicating successful administration of the collagen-containing formulations across all experimental groups (Figure 5B) (blue: collagen, red: cytoplasm, black: cell nuclei). Furthermore, localized submucosal fibrosis with spindle-shaped cells was distinctly observed in the sham group, which served as the secondary atrophic rhinitis model group.

Additionally, the therapeutic effect was compared between the Vehicle and Treatment groups.

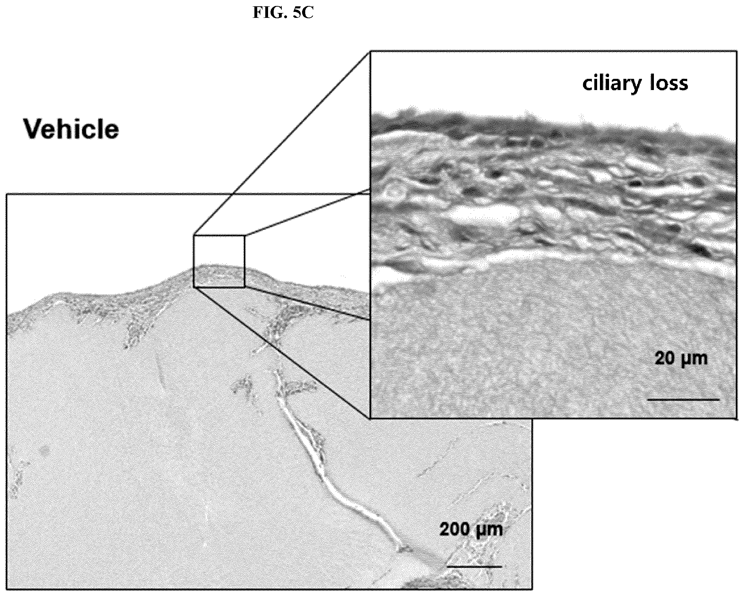

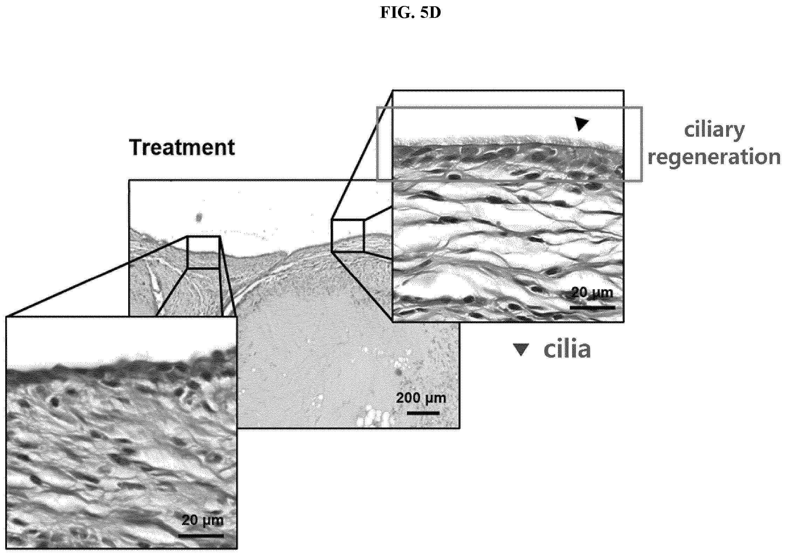

As a result (Figures 5C and 5D), unlike the Sham group and the Vehicle group (which did not exhibit a therapeutic effect with collagen type I alone), the Treatment group displayed enhanced cilia regeneration, facilitated by the injected hNTSCs. Moreover, distinct regions were demarcated based on the presence or absence of nuclei, as detected by hematoxylin staining. Notably, exogenous collagen type I was devoid of nuclei, whereas the submucosal fibrosis region contained nuclei.

To further investigate cilia regeneration, immunohistochemical (IHC) staining for acetylated-α-tubulin, a protein marker of cilia, was performed to confirm the regenerative effect of hNTSCs in tissues.

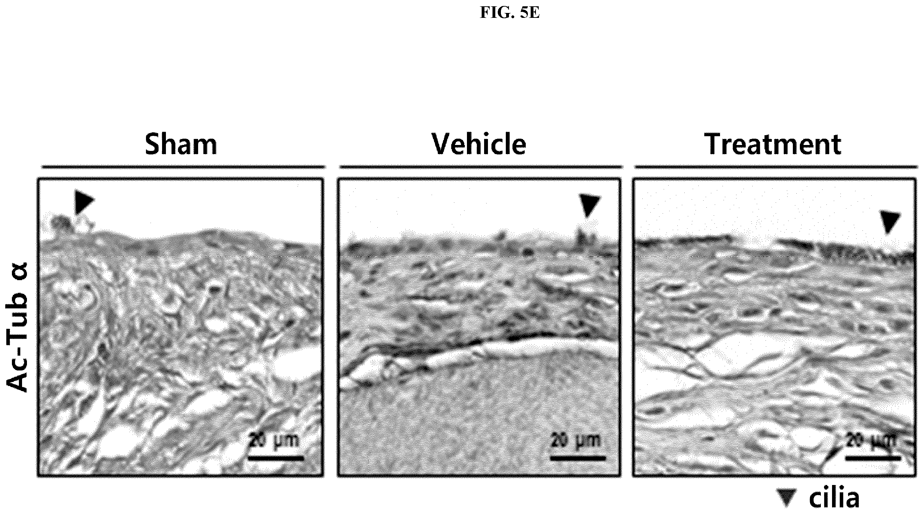

As shown in Figure 5E, the regeneration of cilia in the squamous metaplasia area was confirmed through acetylated-α-tubulin IHC staining (brown: acetylated-α-tubulin, purple: cell nuclei).

Next, the thickness of the nasal mucosa was analyzed using H&E staining after injecting formulations into the Sham, Vehicle, and Treatment groups. Specifically, the nasal mucosa thickness was measured in tissue images, averaged, and quantified.

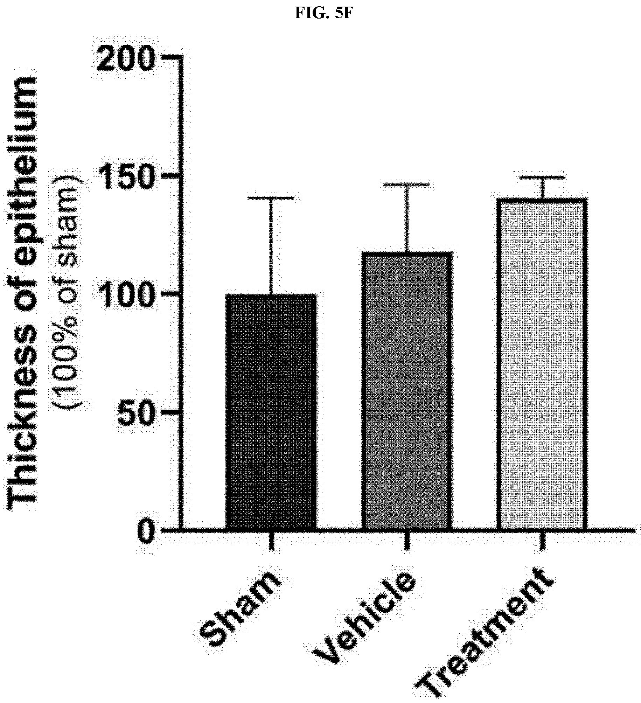

As a result (Figure 5F), when the nasal mucosa thickness of the Sham group was set as 100, the Vehicle and Treatment groups exhibited thicknesses of 117.9% and 140.4%, respectively, confirming an increase in average nasal mucosa thickness. This finding suggests that treatment with human nasal turbinate stem cells in this invention leads to mucosal tissue recovery in secondary atrophic rhinitis.

Lastly, in the Treatment group, the presence of hNTSCs was identified using HuNu (human nuclei) staining, confirming their localization and therapeutic effects.

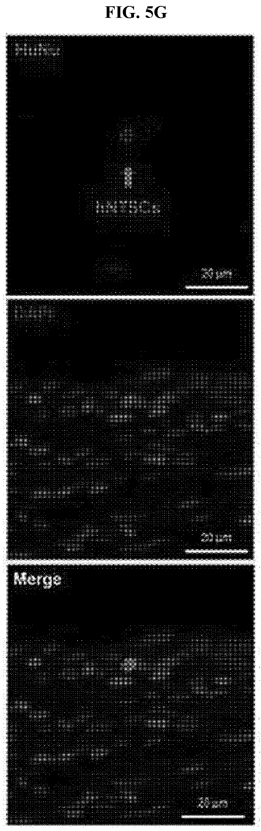

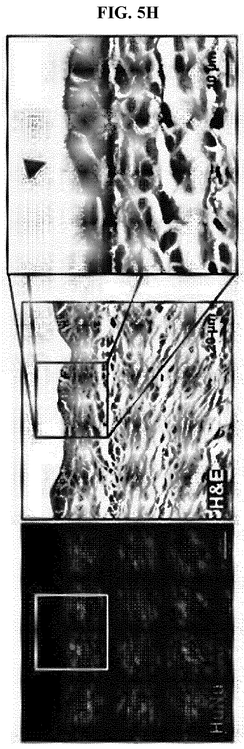

As shown in Figures 5G and 5H, staining for human nuclei (HuNu) demonstrated the migration of hNTSCs into the epithelial layer of the squamous metaplasia region, confirming the long-term retention of injected cells at the injection site even after two months (Figure 5G). Furthermore, localized cilia regeneration was observed adjacent to HuNu-positive regions (Figure 5H).

### Example 4-2. Confirmation of the Therapeutic Effect of Spheroid Injectable Formulation of Human Nasal Turbinate Stem Cells in Secondary Atrophic Rhinitis

First, the transcriptional expression levels of FOXJ1, a cilia cell marker, and MUC5AC, a mucous cell marker, were analyzed in nasal mucosa tissues from the Sham, Vehicle, and Treatment groups at low (low), middle (middle), and high (high) concentrations.

Specifically, RT-qPCR was performed to analyze expression levels (Table 1). After administering the respective formulations to each experimental group, the nasal mucosa was collected, and RNA was extracted using TRIzol. Subsequently, cDNA was synthesized, and the gene expression levels of the markers in the nasal mucosal tissues were confirmed.

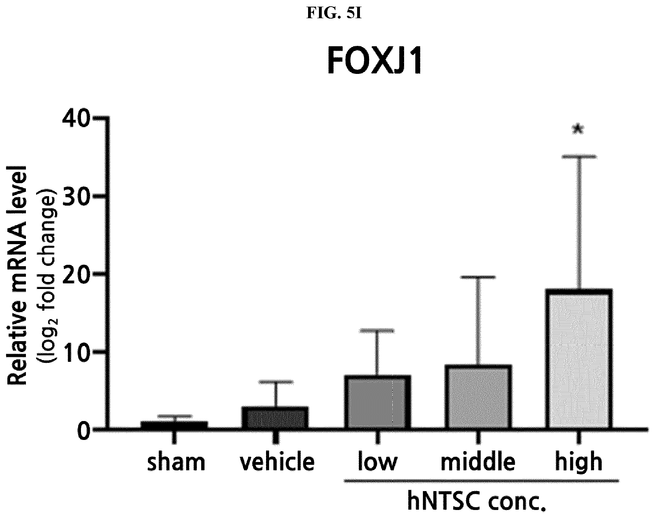

As a result, the mRNA expression level of FOXJ1 was confirmed to be dose-dependent, with the highest levels observed in the high group, followed by the middle, low, vehicle, and sham groups, in that order. In the experimental groups injected with the spheroid injectable formulation of human nasal turbinate stem cells (hNTSCs), the FOXJ1 mRNA expression level was higher compared to the sham and vehicle groups, regardless of concentration. Notably, in the high group, the FOXJ1 mRNA expression level was confirmed to be statistically significant. These experimental results correspond to the cilia regeneration effect observed when hNTSC single cells were injected, suggesting that cilia cell regeneration leads to an increase in cilia count, thereby contributing to overall cilia regeneration (Figure 5I).

Finally, after injecting the treatment group into rabbits, the histology of the nasal mucosal tissue was examined using H&E and Masson's trichrome (MT) staining analysis to confirm the regenerative effect on the nasal mucosa.

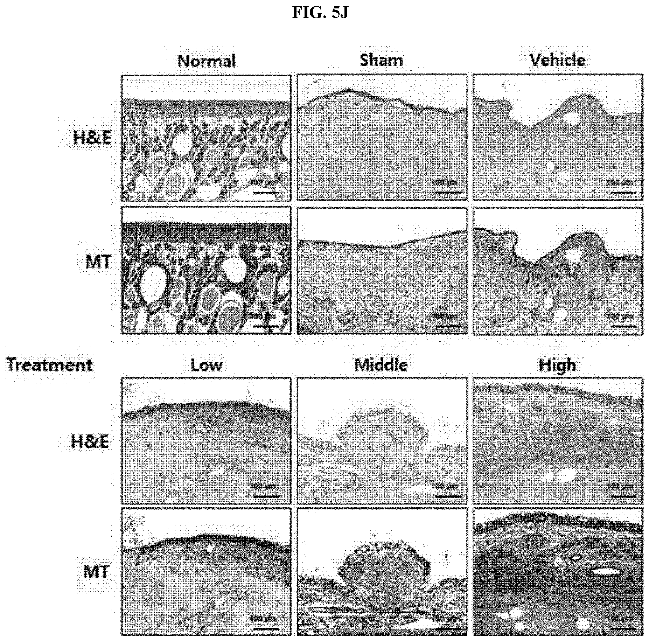

As a result, in the vehicle group, no mucosal regeneration was observed. In contrast, in the treatment groups, cilia formation (low concentration) and both cilia formation and restoration of the pseudostratified columnar epithelium (middle and high concentrations) were observed, indicating a superior level of mucosal regeneration (Figure 5J).

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

### [Industrial Applicability]

The present invention relates to a pharmaceutical composition for the prevention or treatment of secondary atrophic rhinitis, comprising nasal turbinate-derived stem cells as an active ingredient. The study confirmed that treating secondary atrophic rhinitis, induced by turbinate surgery, with nasal turbinate-derived stem cells exhibited superior therapeutic effects. The nasal turbinate-derived stem cells of the present invention not only restored submucosal fibrosis and regenerated cilia but also reversed squamous metaplasia. Furthermore, these stem cells were found to persist long-term at the injection site, significantly enhancing the therapeutic effect. Given these findings, the invention is expected to be highly useful as a preventive or therapeutic agent for secondary atrophic rhinitis, demonstrating industrial applicability.

## Claims

1. A pharmaceutical composition for preventing or treating atrophic rhinitis, comprising nasal turbinate-derived stem cells as an active ingredient.

2. The pharmaceutical composition of Claim 1, wherein the nasal turbinate-derived stem cells are in the form of single cells or spheroids.

3. The pharmaceutical composition of Claim 2, wherein the nasal turbinate-derived stem cells are mixed with type I collagen.

4. The pharmaceutical composition of Claim 1, wherein the nasal turbinate-derived stem cells have the differentiation potential into at least one selected from the group consisting of adipocytes, chondrocytes, and osteocytes.

5. The pharmaceutical composition of Claim 3, wherein the composition comprises nasal turbinate-derived stem cells at a concentration of 1×10⁵ to 1×10¹⁰ cells per mL of type I collagen.

6. The pharmaceutical composition of Claim 1, wherein the composition is a composition for injection.

7. The pharmaceutical composition of Claim 6, wherein the composition is administered via intranasal injection.

8. The pharmaceutical composition of Claim 1, wherein the nasal turbinate-derived stem cells are human nasal turbinate-derived stem cells (hNTSCs).

9. The pharmaceutical composition of Claim 1, wherein the nasal turbinate-derived stem cells are negative for at least one marker selected from the group consisting of IgG-PE, CD14, and CD34, and positive for at least one marker selected from the group consisting of CD73, CD90, and CD105.

10. The pharmaceutical composition of Claim 1, wherein the atrophic rhinitis is secondary atrophic rhinitis.

11. The pharmaceutical composition of Claim 1, wherein the composition is **characterized by** at least one selected from the group consisting of:
a) regenerating ciliated cells and increasing the number of ciliated cells;
b) persisting at the treatment site for an extended period;
c) restoring squamous metaplasia, submucosal fibrosis, and pseudostratified ciliated columnar epithelium;
d) increasing the thickness of the nasal mucosa;
e) increasing the gene expression level of FOXJ1; and
f) regenerating nasal mucosal tissue.

12. A stem cell therapeutic agent for preventing or treating atrophic rhinitis, comprising nasal turbinate-derived stem cells as an active ingredient.

13. A kit for preventing or treating atrophic rhinitis, comprising nasal turbinate-derived stem cells and an instruction manual.

14. A method for preventing or treating secondary atrophic rhinitis, comprising administering a pharmaceutically effective amount of nasal turbinate-derived stem cells or a composition containing the same as an active ingredient, to a subject in need thereof.

15. A use of nasal turbinate-derived stem cells or a composition containing the same as an active ingredient for preventing or treating secondary atrophic rhinitis.

16. A use of nasal turbinate-derived stem cells or a composition containing the same as an active ingredient for the manufacture of a medicament for preventing or treating secondary atrophic rhinitis.
